# FASCICULE DE BREVET EUROPEEN

(11) **EP 1 902 054 B1**
(45) Date de publication et mention de la délivrance du brevet: **18.02.2009**
(21) Numéro de dépôt: 06778710.1
(22) Date de dépôt: 29.06.2006
(51) Int. Cl.: C07D 471/04, A61K 31/519, A61P 35/00

(54) **DERIVES DE PYRIDO[2,3-D]PYRIMIDINE, LEUR PREPARATION, LEUR APPLICATION EN THERAPEUTIQUE**
PYRIDO[2,3-D]PYRIMIDIN-DERIVATE SOWIE DEREN HERSTELLUNG UND THERAPEUTISCHE ANWENDUNG
DERIVATIVES OF PYRIDO[2,3-D]PYRIMIDINE, THE PREPARATION THEREOF AND THE THERAPEUTIC APPLICATION OF THE SAME

(30) Priorité: 01.07.2005 FR 0507032
(43) Date de publication de la demande: 26.03.2008
(60) Demande divisionnaire: 08017944.3
(73) Titulaire: Sanofi-Aventis, 75013 Paris (FR)
(72) Inventeur: CASELLAS, Pierre, F-34090 Montpellier (FR); BOURRIE, Bernard, F-34980 Saint Gely du Fesc (FR); PERREAUT, Pierre, F-34980 Saint Clement de Riviere (FR); MUNEAUX, Claude, F-34270 Les Matelles (FR); JEGHAM, Samir, F-34980 Montperrier (FR)
(74) Mandataire: Le Pennec, Magali
(86) Numéro de dépôt international: PCT/FR2006/001518
(87) Numéro de publication internationale: WO 2007/003765

(56) Documents cités:
- EP-B- 0 790 997
- WO-A-01/55147
- WO-A-01/70741
- WO-A-03/000011
- WO-A-20/04085436
- SCHROEDER M C ET AL: "SOLUBLE 2-SUBSTITUTED AMINOPYRIDOÄ2,3-DÜPYRIMIDIN-7-YL UREAS. STRUCTURE-ACTIVITY RELATIONSHIPS AGAINST SELECTED TYROSINE KINASES AND EXPLORATION OF IN VITRO AND IN VIVO ANTICANCER ACTIVITY" 7 juin 2001 (2001-06-07), JOURNAL OF MEDICINAL CHEMISTRY, AMERICAN CHEMICAL SOCIETY. WASHINGTON, US, PAGE(S) 1915-1926 , XP001152609 ISSN: 0022-2623 le document en entier
- ANDREW M. THOMPSON ET AL.: "Synthesis and Structure-Activity Relationships of soluble 7-Substituted 3-(3,5-Dimethoxyphenyl)-1,6-naphthyridin-2 -amines and Related Ureas as Dual Inhibitors of the Fibroplast Growth Factor Receptor-1 and Vascular Endothelial growth factor Receptor-2 Tyrosine Kinasess" JOURNAL OF MEDICINAL CHEMISTRY., vol. 48, no. 14, 17 juin 2005 (2005-06-17), pages 4628-4653-, XP002372558 USAMERICAN CHEMICAL SOCIETY.

## Description

La présente invention a pour objet des dérivés de pyrido[2,3-d]pyrimidine, leur préparation et leur application en thérapeutique.

### [Art antérieur]

Des composés dérivés de pyndo[2,3-d]pyrimidine sont décrits dans les demandes de brevets WO 01/55147 et WO 03/000011 et dans les brevets EP-B-790 997 et US 6733913**.** Ces composés sont potentiellement utiles pour traiter les troubles de la prolifération cellulaire.
WO 2004/085436 décrit des composés de formule (A) : 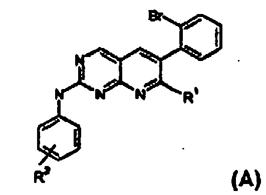 dans laquelle R¹ est une liaison amide -CO-NH- et pas une liaison urée -CO-NH-CO-.

WO 03/000011 décrit des composés de formule (B) : 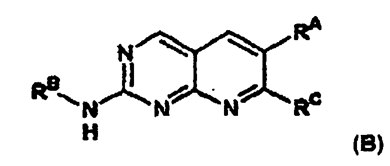 dans laquelle R^{B} est un groupe hétéroaryle masi pas le groupe benzothiadiazole et au moins l'un des groupes R^{A}, R^{B} ou R^{C} comprend un groupe phosphoré.

WO 09/70741 décrit des composés de formule (C) : 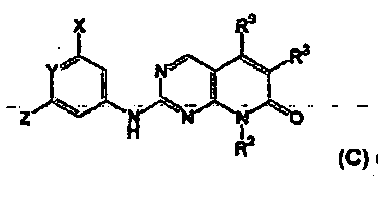 qui ne sont pas des pyrido[2,3-d]pyrimidine.

WO 01/55147 décrit des composés de formule (D) : 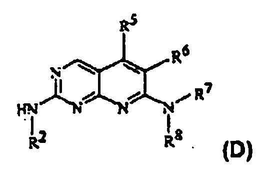 dans laquelle R⁶ n'est pas un groupe aryle et le groupe -NR⁷R⁸ n'est pas une liaison urée.

EP0790997 B1 décrit des composés de formule (E) ou plus spécifiquement de formule (F) : 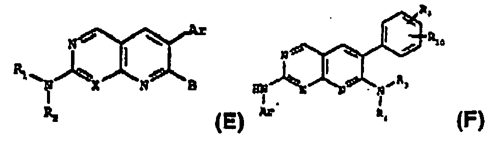 dans lesquelles Ar et Ar' sont des groupes aryle ou hétéroaryle selectionnés dans la liste suivante : phenyl, imidazolyl, pyrrolyl, pyridyl, pyrimidyl, benzimidazolyl, benzothienyl, benzofuranyl, indolyl, pyrazinyl, thazolyl, oxazolyl, isoxazolyl, furanyl, thienyl, naphtyl. Le groupe benzothiadiazole n'est pas mentionné ni suggéré.

L'article J. Med. Chemistry 2001, 44(12), 1915-1926 décrit des pyrido[2,3-d]pyrimidine. Le groupe benzothiadiazole n'est pas mentionné ni suggéré. L'article J. Med. Chem. 2005, 48(14), 4628-4653 ne décrit pas de pyrido[2,3-d]pyrimidine.

### [Invention]

Selon un premier aspect, la présente invention a pour objet des composés répondant à la formule (I) : 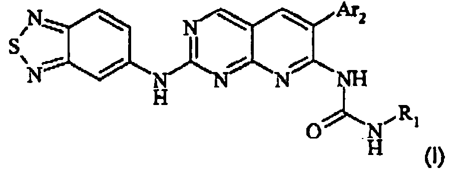 **caractérisés en ce que** Ar₂ et R₁ sont respectivement : 2,6-dichlorophényl et tert-butyl ; ou 2-bcomo-6-chlorophényl et tert-butyl; ou 2,6-dichlorophényl et éthyl ; ou 2,6-dibromophényl et tert-butyl ; ou 2,6-dibromophényl et éthyl ; ou 2,6-dichlorophényl et phényl ; ou 3,5-diméthoxy-phényl et tert-butyl ; ou phényl et tert-butyl ; ou 2,8-diméthylphényl et tert-butyl ; ou 2,8-difluorophényl et tert-butyl ; ou 2,6-dichlorophényl et iso-propyl.

Un composé plus particulièrement préféré selon l'invention est : 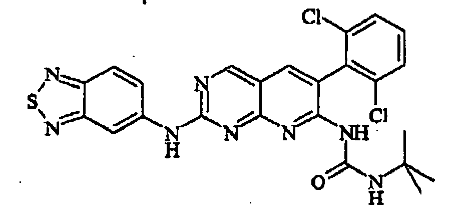 (N-[2-(2,1,3-benzothiadiazol-5-ylamino)-6-(2,6-dichlorophényl)pyrido[2,3-d]pyrimidin-7-yl]-N'-(1,1-diméthyléthyl)-urée)

Un composé conforme à l'invention peut : (i) être sous forme non chirale, ou racémique, ou enrichie en un stéréo-isomère, ou enrichie en un énantiomère ; (ii) être éventuellement salifié, et (iii) être éventuellement hydraté ou solvaté. Les composés de formule (I) peuvent comporter un ou plusieurs atomes de carbone asymétrique. Ils peuvent donc exister sous forme d'énantiomères ou de diastéréoisomères. Ces énantiomères, diastéréoisomères ainsi que leurs mélanges, y compris les mélanges racémiques, font partie de l'invention.

Les composés de formule (I) peuvent exister à l'état de bases ou de sels d'addition à des acides. Lorsque des composés de formule (I) comportent des fonctions acides libres, par exemple carboxylique, sulfonique, phosphonique, ces fonctions acides peuvent être salifiées à l'aide de bases pour former des sels d'addition. De tels sels d'addition font partie de l'invention. Les sels d'addition à des acides ou à des bases sont avantageusement préparés avec, respectivement, des acides ou des bases pharmaceutiquement acceptables, mais les sels d'autres acides ou de bases utiles, par exemple, pour la purification ou l'isolement des composés de formule (I) font également partie de l'invention.

Les composés de formule (I) peuvent également exister sous forme d'hydrates ou de solvates, à savoir sous forme d'associations ou de combinaisons avec une ou plusieurs molécules d'eau ou avec un solvant. De tels hydrates et solvates font également partie de l'invention.

Dans le cadre de la présente invention, on entend par :
- un atome d'halogène : un fluor, un chlore, un brome ou un iode ;
- un groupe alkyle : un groupe aliphatique saturé, linéaire ou ramifié. A titre d'exemple on peut citer les groupes méthyle, éthyle, n-propyle, n-butyle, n-pentyle, n-hexyle, n-heptyle, 1-méthyléthyle, 1-méthylpropyle, 2-méthylpropyle, 1,1-diméthyléthyle, 1-méthylbutyle, 2-méthylbutyle, 3-méthylbutyle, 1,1-diméthylpropyle, 1,2-diméthylpropyle, 2,2-diméthylpropyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1,1,2-triméthylpropyle, 1,2,2-triméthylpropyle, 1-éthyl,1-méthylpropyle, 1-éthyl,2-méthylpropyle, 1-éthylbutyle, 2-éthylbutyle, 1-méthylhexyle, 2-méthylhexyle, 3-méthylhexyle, 4-méthylhexyle, 5-méthylhexyle. 1,1-diméthylpentyle, 1,2-diméthylpentyla, 1,3-diméthylpentyle, 1,4-diméthylpentyle, 2,2-diméthylpentyle, 2,3-diméthylpentyle, 2,4-diméthylpentyle, 3,3-diméthylpentyle, 3,4-diméthylpentyle, 4,4-diméthylpentyle, 1,1,2-triméthylbutyle, 1,1,3-triméthylbutyle, 1,2,2-triméthylbutyle, 1,2,3-triméthylbutyle, 1,3,3-triméthylbutyle, 2,2,3-triméthylbutyle, 2,3,3-triméthylbutyle, 1,1,2,2-tétraméthylpropyle, 1-éthylpentyle, 2-éthylpentyle, 3-éthylpentyle, 1-éthyl,1-méthylbutyle, 1-éthyl,2-méthylbutyle, 1-éthyl,3-méthylbutyle, 2-éthyl,1-méthylbutyle, 2-éthyl,2-méthylbutyle, 2-éthyl,3-méthylbutyle, 1-propylbutyle, 1-(1-méthyléthyl)butyle, 1-(1-méthyléthyl),2-méthylpropyle.

Les composés de formule (I) sont préparés par réaction entre un composé de formule (II) : 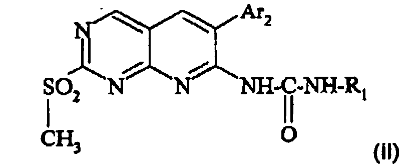 dans laquelle R₁ et Ar₂ sont tels que définis pour (I) et l'amine de formule

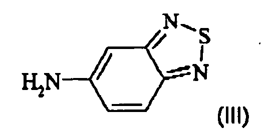

Conformément à l'invention, on peut préparer les composés de formule (I) selon un procédé **caractérisé en ce que** l'on fait réagir :
(i) un composé de formule : 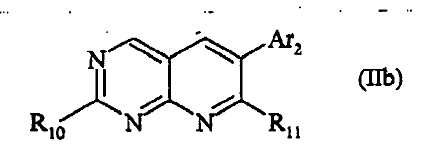 dans laquelle R₁₀ est un groupe partant tel que : (a) halogène, en particulier Cl ou Br, ou (b) alkyl-S(O)ₘ- avec m = 0, 1, ou 2 ; R₁₁ est NHC(O)-NH-R₁; et
(ii) l'amine

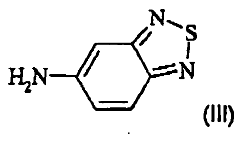

Lorsque R₁₀ est halogène ou alkyl-S(O)ₘ- avec m = 2, La réaction est effectuée dans un solvant, de préférence polaire :
(i) par exemple le tétrahydrofurane, le diméthylsulfoxide ou l'éthanol, éventuellement en présence d'une trace d'acide tel que l'acide chlorhydrique ; ou
(ii) dans le diméthylsulfoxide en présence d'une base forte telle que tBuOK ; à une température comprise entre la température ambiante et la température de reflux du solvant.

Lorsque R₁₀ est alkyl-S(O)ₘ- avec m=0 ou 1, on peut effectuer la réaction avec 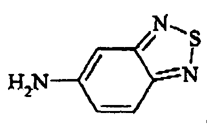 à l'état fondu, de préférence à une température voisine de 200°C, sans catalyseur. Le cas échéant, la fonction amine de (III) est préalablement salifiée ou protégée.

Les composés de formule (II) sont préparés en suivant le mode opératoire décrit dans le brevet européen 790997 et le brevet US 5733913**,** comme décrit dans le Schéma 1 ci-après :

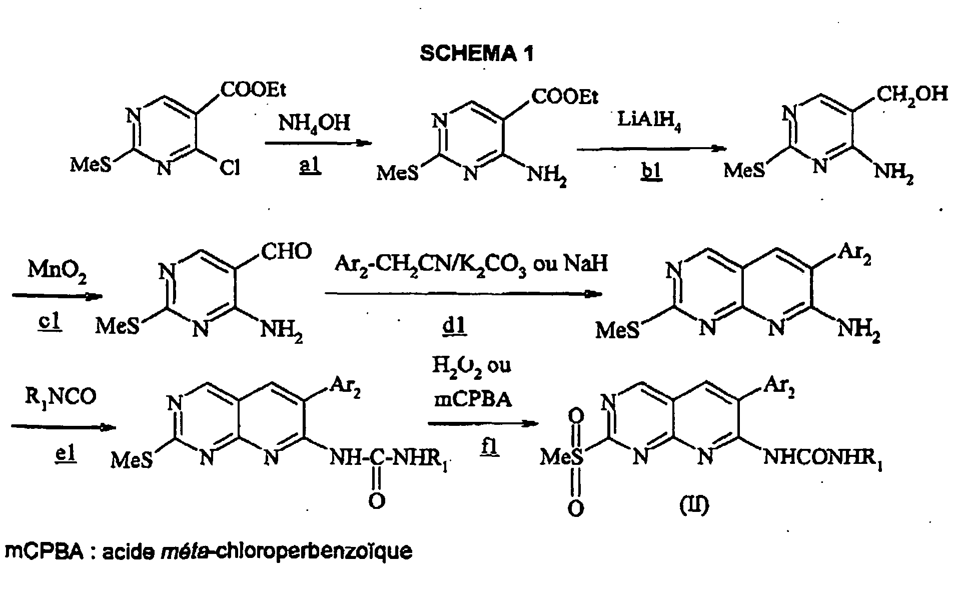

Les composés selon l'invention sont obtenus sous forme racémique ; on peut ensuite préparer les isomères optiquement purs en utilisant des méthodes de dédoublement connues de l'homme de l'art, telle que la cristallisation par formation de sels avec des agents chiraux. On peut également préparer des composés selon l'invention sous forme optiquement pure en utilisant des méthodes de synthèse asymétrique ou stéréospécifique, l'utilisation de techniques chromatographiques utilisant une phase chirale. Par ailleurs, les produits de l'invention peuvent être séparés *via* la formation de diastéréoisomères, leur séparation, puis la décomposition du diastéréoisomère pharmacologiquement utile en son produit actif énantiomériquement pur. Des techniques enzymatiques peuvent aussi être employées. Des techniques séparatives supplémentaires connues peuvent être utilisées. Elles incluent celles divulguées dans: Enantiomers, Racemates, and Resolutions, John Wiley and Sons, New York (1981).

Les exemples suivants décrivent la préparation de certains intermédiaires et de composés conformes à l'invention. Dans les exemples, on utilise les abréviations suivantes :
Boc : *tert*-butoxycarbonyle
BOP : benzotriazol-1-yloxytris(diméthylamino)phosphonium hexafluorophosphate.
THF : tétrahydrofurane
TA : température ambiante
TFA : acide trifluoroacétique
DCM : dichlorométhane
DMSO : diméthylsulfoxyde
DMF: diméthylformamide
MeOH : méthanol.
DCCI : dicyclohexylcarbodiimide
DIPEA :diisopropyléthylamine
KHSO₄ / K₂SO₄ : solution à 5 % de KHSO₄ / K₂SO₄.

Les spectres de résonance magnétique nucléaires (RMN) du proton sont enregistrés à 200 ou 250 MHz dans le DMSO-d₆, sauf indication contraire. Le signal DMSO-d₆ est à 2,5 ppm et sert de référence. Pour l'interprétation des spectres, on utilise les abréviations suivantes ; s : singulet, d : doublet, t : triplet, m : massif, mt : multiplet, se : singulet élargi, dd : doublet de doublet, qd : quadruplet, qt : quintuplet.

F : point de Fusion (en degré Celsius) tel que mesuré sur un appareil Büchi B545 avec un gradient de température de 1°C par minute.

MH+ : Spectre de Masse. Les composés sont analysés par couplage HPLC - UV - MS (chromatographie liquide - détection UV - spectrométrie de masse). L'appareil utilisé, commercialisé par Agilent, est composé d'un chromatographe HP1100 équipé d'un détecteur à barrette de diodes Agilent et d'un spectromètre de masse quadripolaire MSD Quad.

Les conditions analytiques sont les suivantes :
Colonne : Symmetry C18 (50 x 2,1 mm; 3,5 µm)
Eluant A : H₂O + TFA 0,005 % à pH 3,15 ; éluant B : CH₃CN + TFA 0,005 %

| Gradient : | Temps (min) | % B |
|---|---|---|
| | 0 | 0 |
| | 10 | 90 |
| | 15 | 90 |
| | 16 | 0 |
| | 20 | 0 |

Température de la colonne : 30°C ; Débit : 0,4 mL / min; Détection : λ = 210 nm ; tr : temps de rétention ; v : volume.

### Préparation d'un composé de formule (II)

### Préparation 1 : N-(t-Butyl)-N'-[6-(2,6-dichlorophényl)-2-(méthylsulfonyl)pyrido[2,3-d]pyrimidin-7-yl]urée

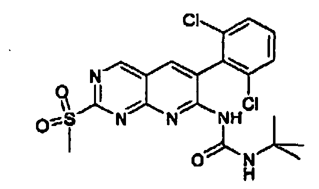

### 1.1 4-Amino-2-(méthylthio)pyrimidine-5-carboxylate d'éthyle

A une suspension de 50,7 g de 4-chloro-2-(méthylthio)pyrimidin-5-carboxylate d'éthyle dans 400 mL d'EtOH on ajoute en 20 minutes, et en maintenant la température vers 20°C, 140 mL d'une solution NH₄OH à 20%. Après 20 heures d'agitation à température ambiante, le milieu réactionnel est concentré sous vide presque à sec, puis le résidu est repris dans 350 mL d'eau, agité 20 minutes, filtré, lavé avec 3x60 mL d'eau puis est séché sous vide en présence de P₂O₅. On obtient un solide blanc, F = 134-135°C, m = 39,9 g.

### 1.2 [4-Amino-2-(méthylthio)pyrimidin-5-yl]méthanol

A 39,68 g d'ester obtenu à l'étape précédente dissous dans 1 litre de THF on ajoute en 45 minutes 210 mL d'une solution 1M en LiAlH₄ dans le THF, en maintenant la température inférieure à 30°C. On agite encore 1 heure puis abaisse la température à 5°C et ajoute successivement goutte à goutte 9 mL d'eau, 6,5 mL de soude 5N puis 32 mL d'eau. Après 10 minutes d'agitation, le solide est filtré puis rincé au THF. Le filtrat est concentré sous vide à sec puis le résidu est redissous dans 600 mL de toluène à ébullition, on filtre rapidement à chaud pour éliminer un peu d'insoluble et laisse refroidir une nuit le filtrat. Les cristaux blancs obtenus sont filtrés, lavés avec un peu de toluène puis d'éther et séchés, F =124-127°C, m = 23,9 g.

### 1.3 4-Amino-2-(méthylthio)pyrimidine-5-carbaldéhyde

A une suspension de 23,8 g de l'alcool obtenu à l'étape précédente dans 1600 mL de chloroforme, on ajoute en 2 minutes 79,5 g de MnO₂ actif et agite 1 nuit à température ambiante : le solide est filtré, lavé par 3x75 mL CHCl₃ et le filtrat concentré sous vide à sec ; le résidu solide blanc est repris dans l'éther, filtré, séché, F =184-186°C, m = 21,05 g.

### 1.4 6-(2,6-Dichlorophényl)-2-(méthylthio)pyrido[2,3-d]pyrimidin-7-amine

A 21 g de l'aldéhyde obtenu à l'étape précédente, dissous dans 240 mL de DMF et refroidis à 5°C on ajoute en 5 minutes 5,47 g de NaH 60% puis en 20 minutes, par petites fractions, 29,05 g de 2,6-dichlorophénylacétonitrile. L'agitation est poursuivie 30 minutes à 5°C puis une nuit à température ambiante. Le milieu réactionnel est refroidi à 5°C et on ajoute 65 mL d'une solution saturée en NH₄Cl puis 500 mL d'un mélange eau/glace; il se forme un précipité rouge qui est filtré, lavé 2 fois à l'eau, essoré au maximum, lavé à l'éther, par 100 mL de chloroforme, puis à l'éther à nouveau ; après séchage on obtient un solide beige, F=250-253°C, m=29,92 g. Les phases éther et chloroforme de lavage sont concentrées à sec, on reprend dans un peu de chloroforme auquel on ajoute de l'éther: on obtient un 2^{ème} jet de 3,15 g, m totale= 33,07 g.

### 1.5 N-(t-Butyl)-N'-[6-(2,6-dichlorophényl)-2-(mémylthio)pyrido[2,3-d]pyrimidin-7-yl]urée

A 29,9 g de l'amine obtenue ci-dessus en solution dans 300 mL de DMF, on ajoute en 10 minutes et en maintenant la température inférieure à 25°C, 4,6 g de NaH à 60% ; on agite encore 20 minutes puis ajoute en 20 minutes 12,2 mL d'isocyanate de tertiobutyle puis agite une nuit. Le milieu réactionnel est versé lentement sur 800 mL d'un mélange eau/glace +100 mL d'HCl 6N ; le précipité formé est filtré, lavé à l'eau, essoré puis agité 1 heure dans 300 mL d'éther, puis filtré, lavé à l'éther et séché. On obtient un solide beige, F = 195-196°C (dec.), m = 28.5 g.

### 1.6 N-(t-Butyl)-N'-[6-(2,6-dichlorophényl)-2-(méthylsulfonyl)pyrido[2,3-d]pyrimidin-7-yl]urée

A 21,95 g d'urée obtenue ci dessus en solution dans 300 mL de chloroforme, on ajoute en 25 minutes, et en maintenant la température inférieure à 25°C, 27 g d'acide métachloroperbenzoïque (77%). Il se forme un précipité. Après 2 heures le milieu réactionnel est dilué par 1 litre de dichlorométhane et on ajoute du Na₂SO₄, puis 14 g de Ca(OH)₂. Après 30 minutes d'agitation le solide est filtré, lavé par du dichlorométhane puis le filtrat concentré à sec. Le résidu est trituré dans 80 mL d'éther à chaud ; on laisse refroidir, puis le solide blanc est filtré, lavé avec de l'éther et séché, F = 138-140°C, m= 20,5 g.

De la même manière que pour le composé décrit à la préparation 1, on peut préparer les composés de formule générale (II) suivants:

**TABLEAU 1**

| **Prép.** | **Ar₂** | **R₁** | **RMN** |
|---|---|---|---|
| **1** | 2,6-dichlorophényl | *tert*-butyl | 1,40: s: 9H; 3,50: s: 3H; 7,50-7,70: m: 3H; 8,55: s: 1H; 9,10: s: 1H; 9,60: s: 1H; 9,95 s: 1H |
| **2** | 2,6-dichlorophényl | phényl | 3,50 ppm : s : 3H ; 7,10 ppm : t : 1H ; 7,40 ppm : t : 2H ; 7,55-7,75 ppm : m : 5H ; 8,60 ppm : s : 1 H ; 9,60 ppm : s : 1H ; 9,80 ppm : s : 1H ; 11,90 ppm : s : 1H. |
| **3** | 3,5-diméthoxyphényl | *tert*-butyl | 1.40 ppm : s : 9H ; 3,50 ppm : s : 3H ; 3,80 ppm : s : 6H ; 6,65-6,80 ppm : mt : 3H ; 7,75 ppm ; s : 1H ; 8,45 ppm : s : 1H ; 9,60 ppm : s : 1 H ; 9,80 ppm : s : 1 H. |
| **4** | 2,6-dichlorophényl | éthyl | 1,20 ppm : t : 3H ; 3,40 ppm : qd : 2H : 3,50 ppm : s : 3H; 7,50 ppm - 7,75 ppm: m: 3H ; 8,55 ppm : s : 1H ; 9,40 ppm : s : 1 H ; 9.60 ppm : s : 1H ; 9,70 ppm : s : 1H. |
| **6** | Phényl | *tert-*butyl | 1,40 ppm : s: 9H ; 3,50 ppm: s: 3H ; 7,60 ppm : se ; 6H ; 8,45 ppm : s : 1 H ; 9,40 ppm : s : 1 H ; 9,80 ppm : s : 1H. |
| **8** | 2,6-dibromophényl | *tert*-butyl | 1,40 ppm : s : 9H ; 3,50 ppm : s : 3H ; 7,40 ppm : t : 1H ; 7,85 ppm : d : 2H : 8.50 ppm : s : 1H ; 9,00 ppm: s : 1H ; 9.60 ppm : s : 1H : 10.00 ppm : s : 1H. |
| **9** | 2-bromo-6-chlorophényl | *tert*-butyt | 1,40 ppm : s : 9H ; 3,45 ppm : s : 3H ; 7,50 ppm : t : 1H ; 7,65 ppm : d : 1H ; 7,80 ppm : d : 1H ; 8,50 ppm : s : 1 H ; 9,00 ppm : s : 1 H ; 9,50 ppm : s : 1 H ; 9,90 ppm : s : 1H. |
| **10** | 2,6-dibromophényl | éthyl | 1,15 ppm : t : 3H : 3.30 ppm : qd : 2H (masqué par DOH) ; 3,50 ppm : s :3H : 7,40 ppm : t : 1 H ; 7,85 ppm : d : 2H ; 8,50 ppm : s : 1 H ; 9,25 ppm : s : 1 H ; 9,60 ppm : s : 1H ; 9,70 ppm : s : 1 H. |
| **14** | 2,6-diméthylphényl | *tert*-butyl | 1,40 ppm : s : 9 H ; 2,00 ppm : s : 6 H ; 3,50 ppm : s : 3 H ; 7,10 ppm : s : 1 H ; 7,25-7,45 : m : 3 H ; 8,45 ppm : s.: 1 H : 9.60 ppm : s : 1 H ; 9.80 ppm : s: 1 H, |
| **15** | 2,8-difluorophényl | *tert*-butyl | 1,40 ppm : s : 9 H ; 3,50 ppm : s : 3 H ; 7,25-7,40 : mt : 2 H ; 7,55-7,70 ppm : mt : 1 H ; 8,65 ppm : s : 1 H ; 9,20 ppm : s : 1 H ; 9,60 ppm : s : 1 H ; 9,75 ppm : s : 1 H. |
| **16** | 2,6-dichlorophényl | *iso*-propyl | 1,20 ppm: d: 6H; 3,50 ppm: s: 3H; 3,85-4.00 ppm: mt: 1H; 7,50-7,70 ppm: m: 3H; 8,50 ppm: s: 1H; 9,25 ppm: s: 1H; 9.65 ppm: s: 1H; 9,75 ppm: se: 1 H. |

L'amine 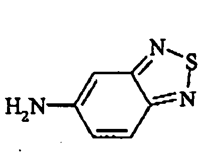 est un produit commercial.

Les numéros des composés exemplifiés renvoient à ceux donnés dans le Tableau 3 ci-après qui illustre les structures chimiques et les propriétés physiques de quelques composés selon l'invention. Lorsqu'ils contiennent un carbone asymétrique, ces composés sont obtenus sous forme racémique.

### Composé N°1: N-[2-(2,1,3-benzothiadiazol-5-ylamino)-6-(2,6-dichlorophényl)pyrido[2,3-d]pyrimidin-7-yl]-N'-(1,1-diméthyléthyl)-urée.

A 3,32 g (20 mmoles) de l'amine 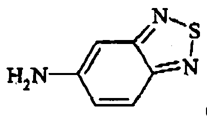 dans 45 mL de DMSO, on ajoute 3,21 g (28,6 mmoles) de t-BuOK en 15 minutes, puis on additionne en 20 minutes 7,71 g (16,5 mmoles) de l'urée de la préparation 1 du tableau 1. 1 g de t-BuOK est rajouté après 2 heures, puis 1 g de t-BuOK est encore ajouté après 2 heures. Après 6 heures de réaction, le milieu réactionnel est dilué par de l'eau glacée, puis extrait par AcOEt. La phase organique est lavée 2 fois par de l'eau, une fois par une solution saturée en NaCl, séchée, concentrée sous pression réduite. Le brut est trituré dans un mélange Et₂O/heptane, le précipité est filtré puis chromatographié sur gel de silice, éluant CHCl₃/AcOEt 88/12 (v/v). On obtient 5 g de produit attendu. MH⁺ = 539.

### Composés N°12-21

Les composés 12-21 sont préparés de la même manière que le composé 1 au départ de l'amine 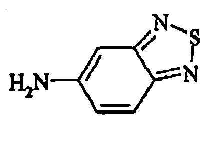 et d'une urée appropriée sélectionnée parmi les produits de formule (II) des préparations du tableau 1.

### Composé N°12 : N-[2-(2,1,3-benzothiadiazol-5-ylamino)-6-(2-bromo-6-chlorophényl)pyrido[2,3-d]pyrimidin-7-yl]-N'-(1,1-diméthyléthyl)-urée.

### Composé N'13: N-[2-(2,1,3-benzothiadiazol-5-ylamino)-6-(2,6-dichlorophényl)pyrido[2,3-d]pyrimidin-7-yl]-N'-éthyl-urée.

### Composé N°14: N-[2-(2,1,3-benzothiadiazol-5-ylamino)-6-(2,6-dibromophényl)pyrido[2,3-d]pyrimidin-7-yl]-N'-(1,1-diméthyléthyl)-urée.

### Composé N°16: N-[2-(2,1,3-benzothiadiazol-5-ylamino)-6-(2,6-dibromophényl)pyrido[2,3-d]pyrimidin-7-yl]-N'-éthyl-urée.

### Composé N°16 : N-[2-(2,1,3-benzothiadiazol-5-ylamino)-6-(2,6-dichlorophényl)pyrido[2,3-d]pyrimidin-7-yl]-N'-phényl-urée

### Composé N°17 : N-[2-(2,1,3-benzothiadiazol-5-ylamino)-6-(3,5-diméthoxyphényl)pyrido[2,3-d]pyrimidin-7-yl]-N'-(1,1-diméthyléthyl)-urée.

### Composé N°18 : N-[2-(2,1,3-benzothiadiazol-5-ylamino)-6-phénylpyrido[2,3-d]pyrimidin-7-yl]-N'-(1,1-diméthyléthyl)-urée.

### Composé N°19 : N-[2-(2,1,3-benzothiadiazol-5-ylamino)-6-(2,6-diméthylphényl)pyrido[2,3-d]pyrimidin-7-yl]-N'-(1,1-iméthyléthyl)-urée.

### Composé N°20: N-[2-(2,1,3-benzothiadiazol-5-ylamino)-6-(2,6-difluorophényl)pyrido[2,3-d]pyrimidin-7-yl]-N'-(1,1-diméthyléthyl)-urée.

### Composé N°21: N-[2-(2,1,3-benzothiadiazol-5-ylamino)-6-(2,6-dichlorophényl)pyrido[2,3-d]pyrimidin-7-yl]-N'-(1-méthyléthyl)-urée.

Le tableau 3 suivant illustre les structures chimiques et les propriétés physiques de quelques exemples selon l'invention.

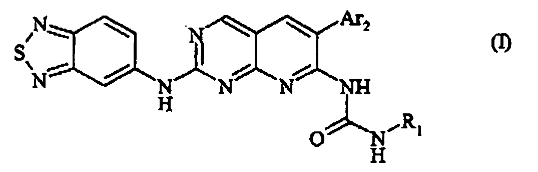

**TABLEAU 3**

| **composé** | **R1** | **Ar2** | **caractérisation RMN** |
|---|---|---|---|
| **1** | *Tert* -butyl | 2,6-Dichlorophényl | 1,50 ppm : s: 9H ; 7,50 - 7,70 ppm : m : 3H ; 7,95 ppm _{:} dd : 2H ; 8,15 ppm ; s ; 1H ; 8,35 ppm ; s ; 1H ; 9,20 ppm : s ; 1 H ; 9,35 pmm : s : 1H ; 10,65 ppm : s : 1H ; 10,75 ppm : s : 1H. |
| **12** | *Tert* -butyl | 2-Bromo-6-chlorophényl | 1,50 ppm : s : 9H ; 7,45 ppm : t : 1H ; 7,65 ppm : d : 1H ; 7,80 ppm : d : 1H ; 8,00 ppm : dd : 2H ; 8,15 ppm : s : 1H ; 8,25 ppm : s : 1H ; 9,20 ppm : s : 1H ; 9,30 ppm : s : 1H ; 10,75 ppm : s : 1 H ; 10,85 ppm : s : 1H. |
| **13** | Ethyl | 2,6-Dichlorophényl | (DMSO + TFA deutéré) 1,15 ppm : t : 3H : 3,35 ppm : qd : 2H ; 7,50-7,70 ppm : m : 3H : 8,00 ppm : dd : 2H ; 8,45 ppm : s : 1H ; 9,00 ppm : s : 1H ; 9.30 ppm : s : 1H. |
| 14 | *Tert*-butyl | 2,6-Dibromophényl | 1,50 ppm : s : 9H ; 7,35 ppm : t : 1H ; 7,80 ppm : d : 2H ; 8,00 ppm : dd : 2H ; 8, 1 5 ppm : s : 2H ; 9, 15 ppm : s : 1H ; 9,30 ppm : s : 1H ; 10,75 ppm : s : 1 H ; 10,85 ppm : s : 1H. |
| **15** | Ethyl | 2,6-Dibromophényl | 1,30 ppm : t : 3H ; 3,30 ppm : qd : 2H ; 7.35 ppm : t : 1H ; 7,80 ppm : d : 2H ; 8,00 ppm : dd : 2H : 8,15 ppm : s : 1H ; 8,50 ppm : s : 1H ; 9,15 ppm : s : 1H ; 9,20 ppm : s : 1H ; 10,20 ppm : s: 1H ; 10,70 ppm : s: 1H. |
| **16** | Phényl | 2,6-Dichlorophényl | 7,15 ppm : t : 1H ; 7,50-7,70 ppm : mt : 5H ; 7,75-8,10 ppm : mt : 4H ; 8,30 ppm : s : 1H ; 9,25 ppm : s : 1H ; 9,35 ppm : s : 1H; 9,50 ppm : s : 1H ; 10,85 ppm : s : 1 H ; 13,30 ppm : s : 1H. |
| **17** | *Tert* -butyl | 3,5-Diméthoxyphényl | 1,50 ppm : s : 9H ; 3,70 ppm : s : 6H ; 6.65 ppm : s : 3H ; 7,30 ppm: s : 1H ; 8,00 ppm : dd : 2H ; 8.20 ppm : s : 1H ; 9,20 ppm : s : 1H ; 9,30 ppm: s : 1H ; 10,50 ppm : s : 1H ; 10,70 ppm : s : 1 H. |
| **18** | *Tert* -butyl | Phényl | 1,50 ppm : s : 9H ; 7,25 ppm : s : 1H ; 7,45-7,65 ppm : m : 5H ; 8,00 ppm : dd : 2H ; 8,20 ppm : s : 1H ; 9,20 ppm : s : 1H ; 9,35 ppm : s : 1H ; 10,50 ppm : s : 1H ; 10,75 ppm : s ; 1 H. |
| **19** | *Tert* -butyl | 2,6-Diméthylphényl | 1,45 ppm : s : 9 H ; 2,00 ppm : s: 6 H ; 6,55 ppm : s : 1 H ; 7,20-7,40 : m : 3 H ; 7,95 ppm : dd : 2 H ; 8,10 ppm : s : 1 H : 9,15 ppm : s : 1 H ; 9,30 ppm : s : 1 H ; 10,45 ppm : s : 1 H ; 10.65 ppm : s : 1 H. |
| **20** | *Tert*-butyl | 2,6-Difluorophényl | 1,45 ppm : s : 9 H ; 7;15-7:30: mt : 2 H ; 7,50-7,70 ppm : mt : 1 H ; 7,95 ppm : dd : 2 H ; 8,25 ppm : s : 1 H ; 8,45 ppm : s : 1 H ; 9,15 ppm : s: 1 H; 9,30 ppm : s : 1 H ; 10,50 ppm: s : 1 H : 10,70 ppm : s : 1 H. |
| **21** | Iso-propyl | 2,6-Dichlorophényl | 1,35 ppm: d: 6H; 3,80-4,00 ppm: mt: 1H; 7,40-7,55 ppm: mt: 1H; 7,60-7,65 ppm: mt: 2H; 7,95 ppm: dd: 2H; 8,15 ppm: s: 1H; 8,60 ppm: s: 1 H; 9,15 ppm: s: 1H; 9,25 ppm: s: 1 H; 10,45 ppm: d: 1 H; 10,75 ppm: s: 1 H. |

Les composés selon l'invention ont fait l'objet d'essais pharmacologiques permettant de déterminer leur activité anticancéreuse.

Les composés de formule (I) selon la présente invention ont été testés in vitro sur un panel de lignées tumorales d'origine humaine provenant :
- de cancer du sein: MDA-MB231 (American Type culture collection, Rockville, Maryland, USA, ATCC-HTB26), MDA-A1 ou MDA-ADR (dite lignée multi-drug résistant MDR, et décrite par E.Collomb et al., dans Cytometry, 12(1):15-25, 1991), et MCF7 (ATCC-HTB22),
- de cancer de la prostate: DU145 (ATCC-HTB81) et PC3 (ATCC-CRL1435),
- de cancer du colon: HCT116 (ATCC-CCL247) et HCT15 (ATCC-CCL225),
- de cancer du poumon: H460 (décrite par Carmichael dans Cancer Research 47 (4):936-942, 1987 et délivré par le National Cancer Institute, Frederick Cancer Research and Development Center, Frederick, Maryland, USA),
- de glioblastome : SF268 (décrite par Westphal dans Biochemical & Biophysical Research Communications 132 (1): 284-289, 1985 et délivré par le National Cancer institute, Frederick Cancer Research and Development Center, Frederick, Maryland, USA),
- de leucémie : CMLT1 (décrite par Kuriyama et al. dans Blood, 74: 1989, 1381-1387, par Soda et al. dans British Journal of Haematology, 59: 1985, 671-679 et par Drexler, dans Leukemia Research, 18: 1994, 919-927 et délivré par la société DSMZ (Deutsche Sammlung von Mikroorganismen und Zellkulturen GmbH) Mascheroder Weg 1b, 38124 Braunschweig, Germany), K-562 (décrite par Lozzio et al., J Natl Cancer Inst 50: 535 (1973), par Lozzio et al., Blood 45: 321 (1975), et délivré par DSMZ n° ACC 10), KG-1 a (décrite par Koeffler et al., Blood 56: 265 (1980), et délivré par DSMZ n°ACC 421), et Kasumi-1 (décrite par Asou et al., Blood 77: 2031 (1991), et délivré par DSMZ n° ACC 220).

La prolifération et la viabilité cellulaire ont été déterminée dans un test utilisant le 3-(4,5-diméthylthiazol-2-yl)-5-(3-eafboxyméthoxyphényl)-2-(4-sulfophényl)-2*H*-tétrazolium (MTS) selon Fujishita T. et al., Oncology 2003, 64 (4), 399-406. Dans ce test, on mesure la capacité mitochondriale des cellules vivantes à transformer le MTS en un composé coloré après 72 heures d'incubation d'un composé de formule (1) selon l'invention. Les concentrations en composé selon l'invention, qui conduisent à 50% de perte de prolifération et de viabilité cellulaire (CI₅₀) sont comprises entre 1 nM et 10 µM, selon la lignée tumorale et le composé testé.

Par exemple, le composé N°1 présente une CI₅₀ de 40 nM sur la lignée K-562, une CI₅₀ de 50 nM sur la lignée KG-1a et une CI₅₀ de 40 nM sur la lignée Kasumi-1. Sur la lignée K-562, le composé N°13 présente une CI₅₀ de 74 nM.

Ainsi, selon la présente invention, il apparaît que les composés de formule (1) entraînent une perte de prolifération et de viabilité des cellules tumorales. Il apparaît donc que les composés selon l'invention ont une activité anticancéreuse et une activité dans le traitement des autres maladies prolifératives telles que le psoriasis, la resténose, l'arthérosclérose, le SIDA par exemple, ainsi que dans les maladies provoquées par la prolifération des cellules du muscle lisse vasculaire et dans la polyarthrite rhumatoïde.

Ainsi selon un autre de ses aspects, l'invention a pour objet des médicaments qui comprennent un composé de formule (1), ou un sel d'addition de ce dernier à un acide pharmaceutiquement acceptable ou encore un hydrate ou un solvate du composé de formule (1). Ces médicaments trouvent leur emploi en thérapeutique, notamment dans le traitement ou la prévention des maladies causées ou exacerbées par la prolifération des cellules et en particulier des cellules tumorales. Un produit conforme à l'invention pourra être utilisé pour la fabrication d'un médicament utile pour traiter un état pathologique, en particulier un cancer.

Comme inhibiteur de la prolifération des cellules tumorales, ces composés sont utiles dans la prévention et le traitement des leucémies, des tumeurs solides à la fois primaires et métastatiques, des carcinomes et cancers, en particulier : cancer du sein ; cancer du poumon ; cancer de l'intestin grêle, cancer du colon et du rectum ; cancer des voies respiratoires, de l'oropharynx et de l'hypopharynx ; cancer de l'oesophage ; cancer du foie, cancer de l'estomac, cancer des canaux biliaires, cancer de la vésicule biliaire, cancer du pancréas : cancers des voies urinaires y compris rein, urothelium et vessie; cancers du tractus génital féminin y compris cancer de l'utérus, du col de l'utérus, des ovaires, chloriocarcinome et trophoblastome; cancers du tractus génital masculin y compris cancer de la prostate, des vésicules séminales, des testicules, tumeurs des cellules germinales; cancers des glandes endocrines y compris cancer de la thyroïde, de l'hypophyse, des glandes surrénales ; cancers de la peau y compris hémangiomes, mélanomes, sarcomes, incluant le sarcome de Kaposi ; tumeurs, du cerveaux _ _ des nerfs, des yeux, des méninges, incluant astrocytomes, gliomes, glioblastomes, rétinoblastomes, neurinomes, neuroblastomes, schwannomes, méningiomes ; tumeurs malignes hématopoïétiques ; leucémies, (Acute Lymphocytic Leukemia (ALL), Acute Myeloid Leukemia (AML), Chronic Myeloid Leukemia (CML), Chronic lymphocytic leukemia (CLL)) chloromes, plasmocytomes, leucémies des cellules T ou B, lymphomes non hodgkiniens ou hodgkiniens, myélomes, hémopathies malignes diverses.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvate dudit composé, ainsi qu'au moins un excipient pharmaceutiquement acceptable. Une composition pharmaceutique peut aussi contenir en outre un autre principe anticancéreux. Lesdits excipients sont choisis selon la forme pharmaceutique et le mode d'administration souhaité, parmi les excipients habituels qui sont connus de l'Homme du métier. Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (1) ci-dessus, ou son sel, solvate ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions. A titre d'exemple une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscarmellose sodique | 6,0 mg |
| Amidon de maïs | 15,0 mg |
| Hydroxypropyl-méthyleelluloss | 2,25 mg |
| Stéarate de magnésium | 3,0 mg |

Les composés de formule (1) ci-dessus peuvent être utilisés à des doses journalières de 0,002 à 2000 mg par kilogramme de poids corporel du mammifère à traiter, de préférence à des doses journalières de 0,1 à 300 mg/kg. Chez l'être humain, la dose peut varier de préférence de 0,02 à 10000 mg par jour, plues Particulièrement de 1 à 3000 mg selon l'âge du sujet à traiter ou le type de traitement : prophylactique ou curatif. Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

Selon la présente Invention, le ou les composés de formule (1) peuvent être administrés en association avec un (ou plusieurs) principe(s) actif(s) anticancéreux, en particulier des composés antitumoraux tels que les agents alkylants tels que les alkylsulfonates (busulfan), la dacarbazine, la procarbazine, les moutardes azotées (chlorméthine, melphalan, chlorambucil), cyclophosphamide, ifosfamide; les nitrosourées tels que la carmustine, la lomustine, la sémustine, la streptozocine; les alcaloïdes antinéoplasiques tels que la vincristine, la vinblastine ; les taxanes tel que le paclitaxel ou le taxotère ; les antibiotiques antinéoplasiques tels que l'actinomycine; les agents intercalants, les antimétabolites antinéoplasiques, les antagonistes des folates, le méthotrexate; les inhibiteurs de la synthèse des purines; les analogues de la purine tels que mercaptopurine, 6-thioguanine; les inhibiteurs de la synthèse des pyrimidines, les inhibiteurs d'aromatase, la capécitabine, les analogues de la pyrimidine tels que fluorouracil, gemcitabine, cytarabine et cytosine arabinoside; le bréquinar ; les inhibiteurs de topoisomérases tels que la camptothécine ou l'étoposide ; les agonistes et antagonistes hormonaux anticancéreux incluant le tamoxifène, les inhibiteurs de kinase, l'imatnib, les inhibiteurs de facteurs de croissance; les antiii1fJammatoires tels que le pentosane polysulfate, les corticostéroïdes, la prednisone, la dexamethasone; les antitopoisomérases tels que l'étoposide, les antracyclines incluant la doxorubicine, la bléomycine, la mitomycine et la méthramycine; les complexes métalliques anticancéreux, les complexes du platine, le cisplatine, le carboplatine, l'oxaliplatine ; l'interféron alpha, le triphénylthiophosphoramide, l'altrétamine ; les agents antiangiogéniques ; la thalidomide , les adjuvants d'immunothérapie ; les vaccins.

Selon la présente invention les composés de formule (I) peuvent également être administrés en association avec un ou plusieurs autres principes actifs utiles dans une des pathologies - indiquées ci-dessus, par exemple un agent anti-émétique, anti-douleur, anti inflammatoire, anti-cachexie. Il est également possible d'associer aux composés de la présente invention un traitement par des radiations. Ces traitements peuvent être administrés simultanément, séparément, séquentiellement. Le traitement sera adapté par le praticien en fonction du malade à traiter.

## Revendications

1. Composé répondant à la formule: 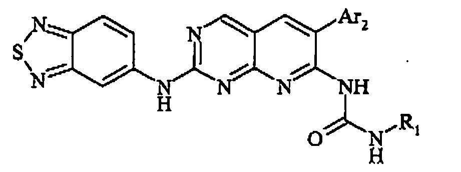 **caractérisé en ce que** Ar₂ et R₁ sont respectivement :
2,6-dichlorophényl et tert-butyl ;
ou 2-bromo-6-chlorophényl et tert-butyl ;
ou 2,6-dichtorophényl et éthyl ;
ou 2,6-dibromophényl et tert-butyl ;
ou 2,6-dibromophényl et éthyl ;
ou 2,6-dichlorophényl et phényl ;
ou 3,5-diméthoxy-phényl et tert-butyl ;
ou phényl et tert-butyl ;
ou 2,6-diméthylphényl et tert-butyl ;
ou 2,6-difluorophényl et tert-butyl ;
ou 2,6-dichlorophényl et iso-propyl.

2. Composé de formule :

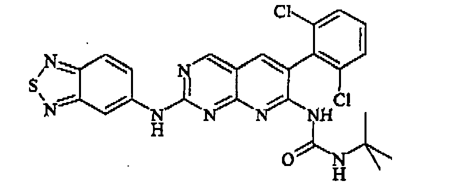

3. Composé selon la revendication 1 ou 2 **caractérisé en ce qu'**il est :
• sous forme non chirale ou racémique ou enrichie en un stéréo-isomère ou enrichie en un énantiomère ;
• **en ce qu'**il est éventuellement salifié ;
• et **en ce qu'**il est éventuellement hydraté ou solvaté.

4. Composition pharmaceutique **caractérisée en ce qu'**elle comprend un composé selon l'une des revendications 1 à 3 ainsi qu'au moins un excipient pharmaceutiquement acceptable.

5. Composition pharmaceutique selon la revendication 4 **caractérisée en ce qu'**elle contient en outre au moins un autre principe actif anticancéreux.

6. Médicament **caractérisé en ce qu'**il comprend un composé selon l'une des revendications 1 à 3 ou un sel d'addition d'un tel composé à un acide pharmaceutiquement acceptable ou encore un hydrate ou solvate d'un tel composé.

7. Composé selon l'une des revendications 1 à 3 en tant qu'anticancéreux.

8. Utilisation d'un composé selon l'une des revendications 1 à 3 pour la préparation d'un médicament destiné au traitement et à la prévention de maladies causées ou exacerbées par la prolifération des cellules.

9. Utilisation selon la revendication 8 pour la prévention et le traitement des leucémies, des tumeurs solides primaires et métastatiques, des carcinomes et cancers.

10. Procédé de préparation d'un composé selon la revendication 1 **caractérisé en ce que** l'on fait réagir sur un composé de formule : 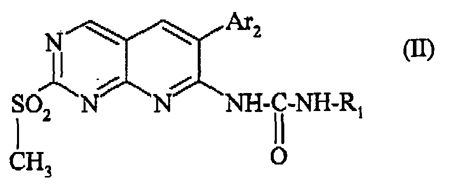 dans laquelle R₁ et Ar₂ sont tels que définis à la revendication 1 une amine de formule

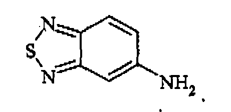

11. Procédé de préparation d'un composé selon la revendication 1 **caractérisé en ce que** l'on fait réagir :
(i) un composé de formulé: 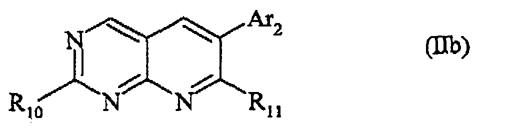 dans laquelle :
- R₁₀ est un groupe partant tel que (a) halogène, en particulier CI ou Br ou (b) alkyl-S(O)ₘ- dans lequel m = 0, 1, ou 2 ;
- R₁₁ est NHC(O)-NH-R₁ ;
(ii) et l'amine de formule 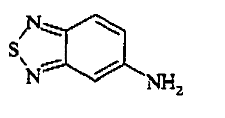
dans lequel :
(a) lorsque R₁₀ est halogène ou alkyl-S(O)ₘ- avec m = 2, la réaction est effectuée dans un solvant, de préférence polaire, à une température comprise entre la température ambiante et la température de reflux du solvant ;
(b) lorsque R10 est alkyl-S(O)m- avec m = 0 ou 1, la réaction est effectuée avec 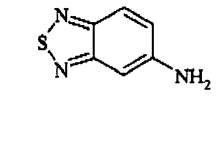 à l'état fondu ; le cas échéant la fonction amine du composé de formule 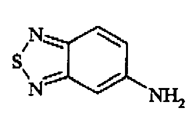 étant préalablement salifiée ou protégée.

## Claims

1. Compound corresponding to the formula: 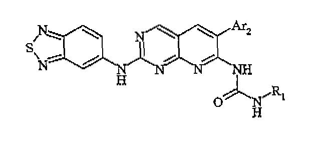 **characterized in that** Ar₂ and R₁ are respectively:
2,6-dichlorophenyl and tert-butyl;
or 2-bromo-6-chlorophenyl and tert-butyl;
or 2,6-dichlorophenyl and ethyl;
or 2,6-dibromophenyl and tert-butyl;
or 2,6-dibromophenyl and ethyl;
or 2,6-dichZorophenyl and phenyl;
or 3,5-dimethoxyphenyl and tert-butyl;
or phenyl and tert-butyl;
or 2,6-dimethylphenyl and tert-butyl;
or 2,6-difluorophenyl and tert-butyl;
or 2,6-dichlorophenyl and isopropyl.

2. Compound of the formula:

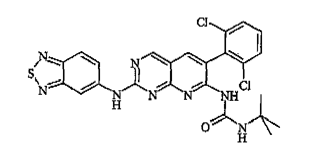

3. Compound according to Claim 1 or 2, **characterized in that** it is in:
• a non-chiral or racemic form or a form enriched in one stereoisomer or enriched in one enantiomer;
• **in that** it is optionally salified;
• and **in that** it is optionally hydrated or solvated.

4. Pharmaceutical composition **characterized in that** it comprises a compound according to any one of Claims 1 to 3 and also at least one pharmaceutically acceptable excipient.

5. Pharmaceutical composition according to Claim 4, **characterized in that** it further comprises at least one other anti-cancer active ingredient.

6. Medicament **characterized in that** it comprises a compound according to any one of Claims 1 to 3 or an addition salt of such a compound with a pharmaceutically acceptable acid, or else a hydrate or a solvate of such a compound.

7. Compound according to any one of Claims 1 to 3 as an anti-cancer agent.

8. Use of a compound according to any one of Claims 1 to 3 for the preparation of a medicament intended for the treatment and for the prevention of diseases caused or exacerbated by cell proliferation.

9. Use according to Claim 8 for the prevention and treatment of leukaemias, primary and metastatic solid tumours, carcinomas and cancers.

10. Method of preparation of a compound according to Claim 1, **characterized in that** a compound of formula: 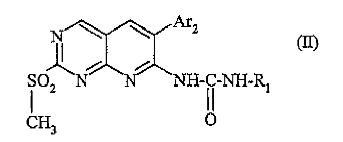 in which R₁ and Ar₂ are as defined in Claim 1 is reacted with an amine of formula

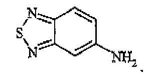

11. Method of preparation of a compound according to Claim 1, **characterized in that** the following are reacted:
(i) a compound of formula: 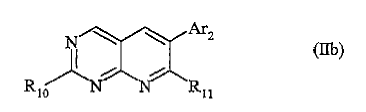 in which:
- R₁₀ is a leaving group such as (a) halogen, in particular Cl or Br, or (b) alkyl-S(O)ₘ- in which m = 0, 1, or 2;
- R₁₁ is NHC(O)-NH-R₁;
(ii) and the amine of formula 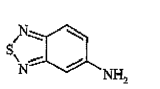
in which:
(a) when R₁₀ is halogen or alkyl-S(O)ₘ- with m = 2, the reaction is carried out in a solvent, preferably a polar solvent, at a temperature between ambient temperature and the reflux temperature of the solvent;
(b) when R₁₀ is alkyl-S(O)ₘ- with m = 0 or 1, the reaction is carried out with 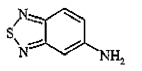 in the molten state; where appropriate, the amine function of the compound of formula 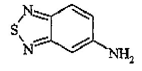 is salified or protected beforehand.

## Patentansprüche

1. verbindung der Formel: 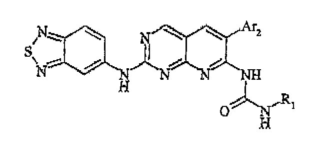 **dadurch gekennzeichnet, daß** Ar₂ und R₁ respektive für
2,6-Dichlorphenyl und tert-Butyl;
oder 2-Brom-6-chlorphenyl und tert-Butyl;
oder 2,6-Dichlorphenyl und Ethyl;
oder 2,6-Dibromphenyl und tert-Butyl;
oder 2,6-Dibromphenyl und Ethyl;
oder 2,6-Dichlorphenyl und Phenyl;
oder 3,5-Dimethoxyphenyl und tert-Butyl;
oder Phenyl und tert-Butyl;
oder 2,6-Dimethylphenyl und tert-Butyl;
oder 2,6-Difluorphenyl und tert-Butyl;
oder 2,6-Dichlorphenyl und Isopropyl
stehen.

2. Verbindung der Formel:

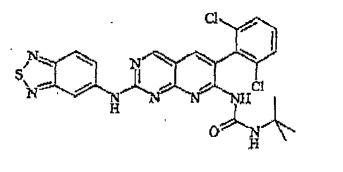

3. Verbindung nach Anspruch 1 oder 2, **dadurch gekennzeichnet, daß** sie:
• in nichtchiraler oder racemischer oder mit einem Stereoisomer angereicherter oder mit einem Enantiomer angereicherter Form vorliegt;
• gegebenenfalls versalzt ist
• und gegebenenfalls hydratisiert oder solvatisiert ist.

4. Pharmazeutische Zusammensetzung, **dadurch gekennzeichnet, daß** sie eine Verbindung nach einem der Ansprüche 1 bis 3 und mindestens einen pharmazeutisch unbedenklichen Hilfsstoff enthält.

5. Pharmazeutische Zusammensetzung nach Anspruch 4, **dadurch gekennzeichnet, daß** sie außerdem mindestens einen anderen Antikrebswirkstoff enthält.

6. Arzneimittel, **dadurch gekennzeichnet, daß** es eine Verbindung nach einem der Ansprüche 1 bis 3 oder ein Additionssalz einer derartigen Verbindung mit einer pharmazeutisch unbedenklichen Säure oder auch ein Hydrat oder Solvat einer derartigen Verbindung enthält.

7. Verbindung nach einem der Ansprüche 1 bis 3 als Antikrebsmittel.

8. Verwendung einer Verbindung nach einem der Ansprüche 1 bis 3 zur Herstellung eines Arzneimittels zur Behandlung und Prävention von Erkrankungen, die durch zellproliferation verursacht oder verschlimmert werden.

9. Verwendung nach Anspruch 8 zur Prävention oder Behandlung von Leukämien, primären und metastatischen soliden Tumoren, Karzinomen und Krebserkrankungen.

10. Verfahren zur Herstellung einer verbindung nach Anspruch 1, **dadurch gekennzeichnet, daß** man eine Verbindung der Formel: 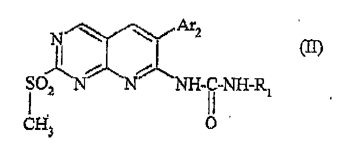 worin R₁ und Ar₂ die in Anspruch 1 angegebene Bedeutung besitzen, mit einem Amin der Formel 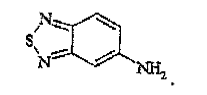 umsetzt.

11. Verfahren zur Herstellung einer Verbindung nach Anspruch 1, **dadurch gekennzeichnet, daß** man:
(i) eine Verbindung der Formel: 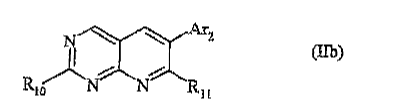 worin:
- R₁₀ für eine Abgangsgruppe wie (a) Halogen, insbesondere Cl oder Br, oder (b) Alkyl-S(O)ₘ-, worin m = 0, 1 oder 2, steht;
- R₁₁ für NHC (O) -NH-R₁ steht;
(ii) und das Amin der Formel 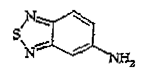 umsetzt, worin:
(a) dann, wenn R₁₀ für Halogen oder Alkyl-S(O)ₘ-mit m = 2 steht, die Umsetzung in einem Lösungsmittel, vorzugsweise einem polaren Lösungsmittel, bei einer Temperatur zwischen Umgebungstemperatur und der Rückflußtemperatur des Lösungsmittels durchgeführt wird;
(b) dann, wenn R₁₀ für Alkyl-S(O)ₘ-mit m = 0 oder 1 steht, die Umsetzung mit 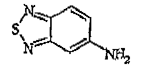 in schmelzflüssigem Zustand durchgeführt wird; wobei die Aminfunktion der Verbindung der Formel 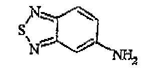 gegebenenfalls vorher versalzt oder geschützt wird.
